Europäisches Patentamt

European Patent Office

Office européen des brevets

(11) Veröffentlichungsnummer: **0 276 385 B1**

(12) **EUROPÄISCHE PATENTSCHRIFT**

(45) Veröffentlichungstag der Patentschrift: **03.06.92**

(51) Int. Cl.⁵: **G10K 9/13, A61B 17/22**

(21) Anmeldenummer: **87116558.5**

(22) Anmeldetag: **10.11.87**

(54) **Schmerzfreie Zerkleinerung von Konkrementen.**

(30) Priorität: **24.01.87 DE 3702120**

(43) Veröffentlichungstag der Anmeldung:
**03.08.88 Patentblatt 88/31**

(45) Bekanntmachung des Hinweises auf die
Patenterteilung:
**03.06.92 Patentblatt 92/23**

(84) Benannte Vertragsstaaten:
**FR GB IT**

(56) Entgegenhaltungen:
**EP-A- 0 081 051**
**KA-B-I- VIT RUM**
**Su-r-g. , G yne**
**US-A- 4 597 388**
**US-A- 4 620 545**

**SIEMENS FORSCHUNGS UND ENTWICK-
LUNGSBERICHTE, Band 15, Nr. 4, 1986, Seiten 187-194, Springer-Verlag, Berlin, DE; H.
REICHENBERGER et al.: "Electromagnetic
acoustic source for the extracorporeal generation of shock waves in lithotripsy"**

(73) Patentinhaber: **DORNIER MEDIZINTECHNIK
GMBH
Postfach 1128
W-8034 Germering 1(DE)**

(72) Erfinder: **Heine, Gerold, Dr.
Reismühlenweg 7
W-7772 Uhldingen-Mühlhofen 1(DE)**
Erfinder: **Marlinghaus, Erst, Dr. Dipl.-Phys.
Kapitän-Wagner-Strasse 34
W-7990 Friedrichshafen 1(DE)**

(74) Vertreter: **Landsmann, Ralf, Dipl.-Ing.
DORNIER GMBH - Patentabteilung - Kleeweg
3
W-7990 Friedrichshafen 1(DE)**

## Beschreibung

Die Erfindung betrifft einen Vorrichtung nach dem Oberbegriff des Anspruchs 1.

Aus der DE-PS 23 51 247 ist es bekannt, Konkremente in Körpern von Lebewesen mit Stosswellen berührungsfrei zu zerkleinern.

Aus der DE-PS 26 50 624 und der DE-PS 32 40 691 ist es bekannt, Konkremente mit Stosswellenimpulsfolgen zu bearbeiten, die so rasch aufeinander folgen, dass mehrere Stosswellen gleichzeitig in einem Konkrement wirken. Dabei werden Resonanzen oder Interferenzen im Konkrement erzeugt, die zu einer schnellen Zerkleinerung führen. Gemäss DE-PS 26 50 624 sind mehrere Energiespeicher vorgesehen, die kurz nacheinander an einer Stosswellenquelle entladen werden. Innerhalb einiger ms werden 5 bis 10 Stoßwellen eingeleitet. Gemäß DE-PS 32 40 691 wird eine einzelne Stoßwelle an einer Platte in einige Teilwellen, die dicht aufeinanderfolgen, zerlegt. Da die Amplitude jeder Teilwelle stets ein Bruchteil der vorhergehenden Welle ist, sind bereits nach einigen Wellen die Amplituden so klein, daß sie keine zerstörende Wirkung mehr haben. Beide Patente geben Lehren zum Erhöhen der Wirksamkeit der Zerkleinerung von Konkrementen. Zur Vermeidung von Schmerzen bei der Behandlung geben beide Patente keine Hinweise.

Aus der US 4,620,545 A ist eine Vorrichtung zur nicht-invasiven Steinzerkleinerung bekannt, bei der eine Folge von Stoßwellen die Steine auflöst. Über den zeitlichen Abstand der einzelnen Stoßwellen sind keine Angaben gemacht.

Aus der US 4,597,388 ist eine Vorrichtung zum Entfernen von Katarakten im menschlichen Auge bekannt, bei der eine Serie elektro-hydraulischer Stoßwellen auf einen Punkt fokussiert werden. Die Wiederholfrequenz kann dabei zwischen 1 und 100 pro Sekunde liegen. Eine Verwendung des Geräts zur Steinzerkleinerung und die Problematik der Schmerzveringerung sind dort nicht angesprochen.

An der klinischen Praxis werden die Patienten teilanästhesiert. Wünschenswert wäre, auf eine Betäubung verzichten zu können, was das Gesamtrisiko der Behandlung verkleinert, den Patienten entlastet und den Krankenhausaufenthalt nochmals verkürzen würde.

Aufgabe der Erfindung ist es, eine Vorrichtung zur Konkrementzerkleinerung anzugeben, bei der Schmerzen soweit vermieden werden, daß die Gabe von Beruhigungs-, Betäubungs- oder Schmerzmitteln reduziert oder ganz weggelassen werden kann.

Diese Aufgabe wird erfindugsgemäß von einer Vorrichtung mit den in Anspruch 1 genannten Merkmalen gelöst. Ausführungen der Erfindung und ein Verfahren sind Gegenstände von Unteransprüchen.

Die Erfindung beruht auf der Erkenntnis, dass das menschliche Schmerzempfinden mehrere Schläge nicht anders spürt, als einen einzigen Schlag, wenn diese nur rasch genug aufeinander folgen. Einmal erregte Schmerzzentren sind für eine gewisse Zeit schmerzunempfindlich. Andererseits wurde festgestellt, dass eine Vielzahl von kurz hinterainanderfolgenden Stosswellen ein Konkrement ebenso zerkleinern, wie die gleiche Zahl von Stosswellen, die in grösseren zeitlichen Abständen folgen. Dies gilt insbesondere auch für Stosswellen, die nicht so rasch aufeinanderfolgen, dass zwei Stosswellen gleichzeitig in einem Konkrement wirken. Die Erfindung schlägt daher eine Vorrichtung vor, die geeignet ist, eine Vielzahl (10 bis 1000) von Stosswellen innerhalb einer Sekunde oder einer kürzeren Zeit in den Körper einzuleiten. Ein Betrieb von weniger als 1 sec Gesamtdauer kann für die Zerstörung ausreichen. Erfindungsgemäss wird dies erreicht durch einen schnellen Schalter zwischen Energiespeicher und Stosswellenquelle, der mit 10 bis 1000 Hz arbeiten kann und einzelne, aufeinanderfolgende Stosswellenfolgen auslösen kann. In einer bevorzugten Ausführungsform ist als Schalter ein Thyratron vorgesehen. Möglich ist auch die Verwendung von Mehrelektrodenfunkenstrecken, Thyristoren, Crossatrons und Pseudospares.

Die Erfindung kann bei verschiedenen Stosswellenquellen realisiert werden. Bei der Erzeugung durch eine Funkenstrecke ebenso wie bei der piezoelektrischen Erzeugung. Anwendbar ist die Erfindung auch bei einer Stosswellenerzeugung mittels einer elektromagnetischen Spule, sofern diese Stosswellenquelle für eine so hohe Frequenz geeignet ist oder bei der Erzeugung durch einen Laser.

Ein erfindungsgemäß benutzter zweiter Mechanismus zur Verringerung von Schmerzen ist die Einstellung eines niedrigeren Fokusdruckes. Wurden bisher stets Drucke über 1 kbar als für die Zertrümmerung von Konkrementen für notwendig gehalten, so hat sich gezeigt, dass die Zerkleinerung auch schon mit Drucken von 300 bar bis 1kbar im Fokus möglich ist. Dies kann erreicht werden durch eine niedrigere Spannung an der Stosswellenquelle, besser ist jedoch eine erniedrigte Kapazität der Energiequelle, was zu einem niedrigeren Stromfluss führt, wenn die Spannung gleich hoch bleibt. Bei Unterwasserfunkenstrecken sollte eine Spannung von 14 bis 30 kV aber nicht unterschritten werden, damit sicher Stosswellen ausgelöst werden können.

Ein dritter Mechanismus zur Vermeidung von Schmerzen ist die Verringerung des Druckes an der Einkoppelstelle auf der Haut des Patienten. Dies kann z.B. durch eine Vergrösserung der Apertur der Stosswellenquelle erreicht werden, da diese Vergrösserung zu einer Erniedrigung des Flächendruckes fuhrt. Realisierbar ist dies bei einer Stosswellenerzeugung durch Unterwasserfunkenstrecke durch einen Reflektor mit vergrösserter Apertur. Bei der Erzeugung mit Piezoelementen oder mit elektromagnetischen Spulen durch eine grösseren Durchmesser der Stosswellenquelle.

**Patentansprüche**

1. Berührungslose Zerkleinerungsvorrichtung von Konkrementen in Körpern von Lebewesen mit einer Stoßwellenquelle, einem Energiespeicher und einem dazwischengeschalteten Schalter, **dadurch gekennzeichnet,** daß die Vorrichtung derart ausgestaltet ist, daß eine Folge von Stoßwellen mit einer Wiederholfrequenz im Bereich von 10 bis 1000 Hz, einem Druck von maximal 0,3 bis 3 kbar im Fokus und von maximal 50 bar an der Einkoppelstelle in den Körper erzeugt werden.

2. Vorrichtung nach Anspruch 1, **dadurch gekennzeichnet,** daß der Schalter ein Thyratron, eine Funkenstrecke, eine Mehrelektrodenfunkenstrecke, ein Thyristor, ein Crossatron oder ein Pseudospare ist.

3. Vorrichtung nach Anspruch 1, **gekennzeichnet durch** einen als Energiespeicher dienenden Kondensator mit 20 bis 60 nF Kapazität, bevorzugt mit 40 nF Kapazität.

4. Berührungslose Zerkleinerungsverfahren von Konkrementen in Kör pern von Lebewesen durch Stoßwellen, **gekennzeichnet durch** die Erzeugung einer Folge von Stoßwellen mit einer Wiederholfrequenz im Bereich von 10 bis 1000 Hz und einem Druck von maximal 0,3 bis 3 kbar im Fokus und von maximal 50 bar an der Einkoppelstelle in den Körper.

**Claims**

1. Contactless communition device for concretions in the bodies of living beings, having a shock wave source, an energy store and a switching device switched therebetween, characterised in that the device is formed in such a way that a series of shock waves are generated with a repetition frequency of in the vicinity of 10 to 1000 Hz, a maximum pressure of 0.3 to 3 kbar at the focus, and of 50 bar at the location of coupling into the body.

2. Device according to claim 1, characterised in that the switching device is thyratron, a spark gap, a multi-electrode spark gap, a thyristor, a crossatron or a pseudospare.

3. Device according to claim 1, characterised by a capacitor acting as an energy store, with a capacitance of 20 to 60 nF, preferably of 40 nF.

4. Contactless communition method for concretions in the bodies of living beings by means of shock waves, characterised by the generation of a series of shock waves with a repetition frequency in the vicinity of 10 to 1000 Hz and a maximum pressure of 0.3 to 3 kbar at the focus point and of 50 bar at the location of coupling into the body.

**Revendications**

1. Dispositif de désintégration sans contact de concrétions à l'intérieur des corps d'êtres vivants, comprenant une source d'ondes de choc, un accumulateur d'énergie et un interrupteur intercalé entre les deux, **caractérisé en ce** que le dispositif est conformé de manière qu'une succession d'ondes de choc est générée avec une fréquence de répétition de l'ordre de 10 à 1000 Hz, une pression maximale de 0,3 à 3 kbars au foyer et au maximum de 50 bars au point de couplage dans le corps.

2. Dispositif selon la revendication 1, caractérisé en ce que l'interrupteur est un thyratron, un éclateur, un éclateur à plusieurs électrodes, un thyristor, un crossatron ou un pseudospare.

3. Dispositif selon la revendication 1, caractérisé en ce qu il comprend un condensateur d'une capacité de 20 à 60 nF, de préférence d'une capacité de 40 nF, servant d'accumulateur d'énergie.

4. Procédé de désintégration sans contact de concrétions à l'intérieur des corps d'êtres vivants au moyen d'ondes de choc, **caractérisé en ce** qu'il consiste à générer une succession d'ondes de choc avec une fréquence de répétition de l'ordre de 10 à 1000 Hz et une pression maximale de 0,3 à 3 kbars au foyer et au maximum de 50 bars au point de couplage dans le corps.